# NEUE EUROPÄISCHE PATENTSCHRIFT

(11) **EP 1 855 660 B2**
(45) Veröffentlichungstag und Bekanntmachung des Hinweises auf die Entscheidung über den Einspruch: **16.10.2019**
(45) Hinweis auf die Patenterteilung: 27.07.2011
(21) Anmeldenummer: 06723143.1
(22) Anmeldetag: 28.02.2006
(51) Int. Cl.: A61K 9/70, A61K 47/32, A61K 47/34, A61K 31/465

(54) **FASERFREIES TRANSDERMALES THERAPEUTISCHES SYSTEM UND VERFAHREN ZU SEINER HERSTELLUNG**
NON-FIBROUS TRANSDERMAL THERAPEUTIC SYSTEM AND METHOD FOR ITS PRODUCTION
SYSTEME THERAPEUTIQUE TRANSDERMIQUE EXEMPT DE FIBRES ET SON PROCEDE DE PRODUCTION

(30) Priorität: 07.03.2005 DE 102005010255
(43) Veröffentlichungstag der Anmeldung: 21.11.2007
(73) Patentinhaber: LTS LOHMANN Therapie-Systeme AG, 56626 Andernach (DE)
(72) Erfinder: MECONI, Reinhold, 56567 Neuwied (DE); SCHUMANN, Klaus, 56567 Neuwied (DE)
(74) Vertreter: Schweitzer, Klaus
(86) Internationale Anmeldenummer: PCT/EP2006/001840
(87) Internationale Veröffentlichungsnummer: WO 2006/094681

(56) Entgegenhaltungen:
- EP-A- 0 261 402
- EP-A- 0 303 025
- WO-A-00/37058
- WO-A-2004/091590
- WO-A1-00/37058
- DE-A1- 4 400 769
- DE-A1- 10 110 391
- US-A- 5 626 866

## Beschreibung

Die vorliegende Erfindung betrifft Verfahren zur Herstellung von transdermalen therapeutischen Systemen (TTS).

Die Erfindung betrifft Verfahren zur Herstellung transdermaler therapeutischer Systeme, bei denen die Systeme unter Vermeidung faserförmiger Bestandteile hergestellt und mittels eines Verteilerplatten-Druckverfahrens mit Wirkstoff beladen werden.

Transdermale therapeutische Systeme sind Systeme zur kontrollierten Verabreichung von pharmazeutischen Wirkstoffen über die Haut. Sie werden seit längerem zur Behandlung unterschiedlicher Krankheiten, körperlicher wie geistiger Funktionsstörungen, Beschwerden sowie Unpässlichkeiten eingesetzt. Bei transdermalen therapeutischen Systemen handelt es sich um schichtförmig aufgebaute Erzeugnisse in Form von Pflastern, die eine wirkstoffundurchlässige Rückschicht, mindestens eine wirkstoffhaltige Reservoir- oder Matrixschicht, gegebenenfalls eine die Geschwindigkeit der Wirkstofffreisetzung kontrollierende Membran und eine ablösbare Schutzschicht, die vor Gebrauch des TTS von diesem abgezogen wird, umfassen.

Zur Befestigung eines transdermalen therapeutischen Systems auf der Haut sowie zur Gewährleistung der kontrollierten Verabreichung des Wirkstoffs ist das TTS mit einer haftklebenden Schicht versehen. Diese haftklebende Schicht kann mit der wirkstoffhaltigen Matrixschicht oder der hautseitigen wirkstoffhaltigen Schicht identisch sein, kann aber auch zusätzlich vorhanden sein, sofern die (hautseitige) wirkstoffhaltige Schicht oder die optional vorhandene Membran nicht haftklebend ist.

Gebräuchlich sind transdermale therapeutische Systeme, die ein wirkstoffhaltiges Reservoir aufweisen, das aus einem Vliesstoff oder einem Papier aufgebaut ist, d. h. aus einem faserförmigen Material. Als Beispiel für derartige transdermale therapeutische Systeme kann das Nikotin enthaltende TTS Nicotinell® genannt werden. Die Herstellung derartiger vliesstoffhaltiger TTS kann beispielsweise nach dem in EP 0 261 402 A1 beschriebenen Verfahren erfolgen.

Die Rückschicht eines TTS muss für den im TTS enthaltenen Wirkstoff undurchlässig sein, um ein unerwünschtes Austreten des Wirkstoffs aus der Seite des TTS, die der Haut abgewandt ist, zu verhindem. Hierfür werden insbesondere Metallfollen, spezielle Kunststofffolien sowie Verbundlaminate dieser Werkstoffe eingesetzt. Am gebräuchlichsten sind Verbundlaminate aus Aluminium und Kunststoffmaterialien wie Polyethylenterephthalat. Der Vorteil dieser Verbundlaminate liegt darin, dass Aluminumfolien kostengünstig herzustellen und gegenüber nahezu allen pharmazeutischen Wirkstoffen undurchlässig sind. Zudem sind Aluminiumfolien lichtundurchlässig, was gerade bei lichtempfindlichen Wirkstoffen den Vorteil eines zuverlässigen Schutzes vor Licht bietet.

Ein Nachteil transdermaler therapeutischer Systeme, die mit einer Rückschicht ausgestattet sind, welche eine Aluminiumfolie und/oder farblich gestaltete Kunststofffolie umfasst, besteht darin, dass sie auf der Haut des Anwenders stets optisch auffallen. Selbst wenn das auf der Haut des Anwenders haftende TTS durch das Tragen von Kleidung verdeckt werden kann, besteht die Gefahr, dass die Akzeptanz von TTS mit einer derartigen Rückschicht beim Anwender sehr gering ist.

Daher sind in jüngerer Zeit TTS erhältlich, die eine durchsichtige Rückschicht besitzen und bei denen auch die weiteren Schichten lichtdurchlässig sind. Bei ihrer Anwendung sind diese TTS gegenüber der Haut, auf der sie haften, fast unsichtbar, da die natürliche Hautfarbe des Anwenders durch das TTS hindurch sichtbar ist.

Ein Vorfahren zur Herstellung transdermaler therapeutischer Systeme mit einer transparenten Rückschicht wird im US Patent 5,626,866 beschrieben. Dabei wird ein wirkstoffhaltiges Gel aus dem Wirkstoff, einer Mischung verschiedener Permeationsenhancer und einem Gelbildner hergestellt und zwischen zwei Klebstoffschichten extrudiert. Aus dem so erzeugten wirkstoffhaltigen Laminat werden anschließend einzelne Wirkstoff-Pflaster ausgestanzt.

In WO 00/37058 A1 werden transparente Nikotin-TTS offenbart, die einen Opazitätsindex von 17,04% bzw. 19,66 % aufweisen. Ihre Herstellung erfolgt gemäß dem in US Patent 5,004,610 beschriebenen Verfahren. Als transparente Rückschichten werden Scotchpak® 1220 der Fa. 3M oder Saranex® der Firma Dow Chemical verwendet. Darüberhinaus kann als transparente Rückschicht auch ein Film aus Baranes® verwendet werden, einem Material aus einem Pfropf-Copolymer aus etwa 73 bis 77 % Acrylonitril und etwa 27 bis 23 % Methacrylat, das in Gegenwart von 8 bis 18 Gew.-% Butadien/Acrylnitril-Copolymeren mit etwa 70 Gew.-% Polymereinheiten auf Butadienbasis hergestellt wird.

Zur Herstellung von Nikotin enthaltenden TTS kann das Tampondruckverfahren verwendet werden, das aus EP 0 303 025 A1 bekannt ist. Bei diesem Verfahren werden mittels eines eiförmigen Silikon-Schaumgummitampons mit einer Shore-Härte von 6 und einer 50 Gew.-%-igen Lösung von Nikotin in Miglyol®812 Lösungsmengen von 91 mg auf eine Acrylat-Haftkleberoberfläche gedruckt. Das so bedruckte Substrat wird abschließend mit einer aluminiumbedampften Polyesterfolie, also einer nicht-transparenten Schicht, kaschiert.

Aus DE 44 00 769 A1 ist schließlich ein Verfahren zur Erstellung flächiger Darreichungsformen bekannt, bei dem eine fließfähige Zubereitung mit großer Genauigkeit durch eine mit mindestens einem Durchlass versehene Verteilerplatte einer Auftragsvorrichtung auf ein mit dieser in Kontakt bringbares, vliesförmiges Substrat flächenmäßig definiert übertragen wird.

Aufgabe der Erfindung war es, ein Verfahren zur Erstellung eines transdermalen therapeutischen Systems bereitzustellen, bei dem die beschriebenen Nachteile vermieden werden.

Die Aufgabe wird durch ein Verfahren nach Anspruch 1 gelöst. Das hergestellte TTS umfasst eine wirkstoffundurchlässige, lichtdurchlässige Rückschicht, zumindest eine lichtdurchlässige Haftkleberschicht, auf die eine wirkstoffhaltige Zubereitung aufgebracht wurde, und eine ablösbare Schutzschicht. Es zeichnet sich dadurch aus, dass es keine faserförmigen Bestandteile aufweist. Auch optional vorhandene weitere Reservoir- oder Matrixschichten sind ebenfalls faserfrei und lichtdurchlässig. Dieses TTS besteht somit ausschließlich aus lichtdurchläßigen Schichten und ist frei von faserförmigen Bestandteilen.

Unter einer lichtdurchlässigen Schicht wird eine transparente (= durchsichtige) oder transluzente (= durchscheinende) Schicht verstanden. Eine transparente Schicht lässt Licht fast unbehindert passieren, wogegen eine transluzente Schicht den größten Teil des Lichtes passieren lässt, dabei das Licht aber diffus streut. Die Schichten einer bevorzugten Ausführungsform des TTS sind klar (ganz) durchsichtig. Zumindest eine der Schichten des TTS kann jedoch auch getönt oder durch Anteile von organischen und/oder anorganischen Farbpigmenten gefärbt sein, so dass der Grad ihrer Transparenz von der Wellenlänge des Lichts abhängt. Auf diese Weise lassen sich ganz oder teilweise lichtundurchlässige TTS bereitstellen.

Unter einem faserförmigen Bestandteil im Sinne der vorliegenden Beschreibung sind verhältnisnmäßig lange, dünne und flexible Gebilde aus natürlichem oder synthetischem Material zu verstehen. Zu den faserförmigen Bestandteilen aus natürlichen Materialien zählen beispielsweise Pflanzenfasern, tierische Fasern sowie Mineralfasern. Pflanzenfasern wie Bast, Baumwolle, Hanf, Kokos, Leinen, Kapok oder Ramie bestehen im Allgemeinen aus Zellulose. Zu den tierischen Fasern gehören Seide und Haare (Wolle). Eine natürlich vorkommende Mineralfaser ist Asbest. Zu den synthetischen Fasern zählen Fasern aus Perlon, Nylon, aber auch Kunstseide, Glasfasern und Kohlenstofffasern. Mehrere Fasern bilden gemeinsam größere Strukturen. So können Textilfasem gemeinsam einen Faden, ein Seil oder einen Stoff bilden. Zellulosefasem werden z. B. zur Papier- und Textilherstellung verwendet. Auf dem Gebiet der transdermalen therapeutischen Systeme werden Fasem hauptsächlich in Form von Vliesstoffen, Papier, Langfaserpapier und textilen Flächenmaterialien mit Stütz- und/ oder Verteilungsfunktion.oder mit Reservoirfunktion verwendet.

Die Haftkleberschicht und/oder optional vorhandene(n) Reservoir- bzw. Matrixschicht(en) des TTS kann/können ein Material umfassen, das aus der Gruppe ausgewählt ist, die aus haftklebenden Polymeren auf Basis von Acrylsäure und/oder Methacrylsäure sowie deren Ester, Polyacrylaten, Isobutylen, Polyvinylacetat, Ethylen-Vinylacetat, natürlichen und/oder synthetischen Kautschuken, beispielsweise Acrylnitril-Butadien-Kautschuk, Butylkautschuk oder Neoprenkautschuk, Styrol-Dien-Copolymeren wie Styrol-Butadien-Blockcopolymeren und Heißschmelzklebem besteht, oder das auf Basis von haftklebenden Silikonpolymeren oder Polysiloxane hergestellt ist.

Vorzugsweise ist das Material, welches die Haftkleberschicht umfasst, aus der Gruppe ausgewählt, die kationische Copolymere auf Basis von Dimethylaminoethylmethacrylat und neutralen Methacrylsäureestem, beispielsweise Eudragil® E 100, und neutrale Copolymere auf Basis von Butylmethacrylat und Methylmethacrylaten, zum Beispiel. Plastoid® B, umfaßt.

Für durchsichtige oder transluzente TTS muss die Haftkleberschicht des TTS lichtdurchlässig sein. Die Haftkleberschicht ist vorzugsweise klar durchsichtig, kann aber auch getönt oder gefärbt sein.

Die mit der Haftkleberschicht bzw. einer optional zusätzlich vorhandenen Reservoir- bzw. Matrixschicht verbunden Rückschicht ist undurchlässig für den mit dem jeweiligen TTS zu verabreichenden Wirkstoff und besitzt okklusiven Charakter. Sie ist frei von faserförmigen Bestandteilen. Bei einer besonders bevorzugten Ausführungsform ist die Rückschicht farblos, d. h. klar durchsichtig. Die lichtdurchlässige Rückschicht kann aber auch getönt oder durch Anteile von organischen und/oder anorganischen Farbpigmenten gefärbt sein,

Für die Rückschicht können beliebige Materialien eingesetzt werden, die die vorgenannten Bedingungen erfüllen. Derartige Materialien können beispielsweise Polymere aus der Gruppe sein, die Polyethylenterephthalat, weichgemachte Vinylacetat-Vinylchlorid-Co-polymerisate, Nylon, Ethylen-Vinylacetat-Copolymerisate, weichgemachte Polyvinylchlorid, Polyurethan, Polyvinylidenchlorid, Polypropylen, Polyethylen oder Polyamid umfasst.

Das transdermale therapeutische System enthält mindestens einen pharmazeutischen Wirkstoff, der über die Haut an eine Person abgegeben werden kann. Als Wirkstoff sind prinzipiell alle transdermal applizierbaren pharmazeutischen Wirkstoffe geeignet, die als Einzelwirkstoffe oder in Kombinationen miteinander in einem TTS enthalten sein und transdermal verabreicht werden können. Der Wirkstoff kann bei Raumtemperatur ein Feststoff, aber auch flüssig sein. Sowohl nicht-flüchtige Wirkstoffe wie Fentanyl, aber auch flüchtige Wirkstoffe wie Nikotin eignen sich, um mit dem TTS verabreicht zu werden.

Weitere Wirkstoffe, die in dem TTS enthalten sein können, sind beispielsweise Alprostadil, Buprenorphin, Clonidin, Dexmethason, Dextroamphetamin, Diclofenac, Dihydrotestosteron, Estradiol (auch in Kombination mit androgenen oder progestinen Wirkstoffen), Fentanyl, Flurbiprofen, Lidocain, Methylphenidat, Nitroglycerin, Rotigotin, Salicylsäure, Scopolamin, Testosteron und Tulobuterol.

Das TTS wird unter Vermeidung faserförmiger Bestandteile hergestellt, indem der Wirkstoff mittels eines Verteilerplatten-Druckverfahrens auf die faserfreie Haftkleberschicht aufgebracht wird.

Bei dem Druckverfahren, das überraschenderweise zur Herstellung der transdermalen therapeutischen Systeme geeignet ist, wird die Wirkstoff enthaltende Zubereitung durch eine mit mindestens einem Durchlass versehene Verteilerplatte einer Auftragsvorrichtung auf die faserfreie Haftkleberschicht übertragen.

Zur Herstellung des TTS kann zunächst eine faserfreie, vorzugsweise durchsichtige Haftkleberschicht auf einer wirkstoffundurchlässigen Trägerschicht hergestellt werden. Auf diese Haftkleberschicht werden einzeln dosierte Portionen einer wirkstoffhaltigen Zubereitung aufgetragen.

In einem weiteren Schritt wird die Rückschicht so auf die mit der wirkstoffhaltigen Zubereitung versehene Haftkleberschicht aufgebracht, dass die wirkstoffhaltige Zubereitung nicht mehr auf dieser Seite des so entstandenen Verbundlaminats austreten kann. Aus dem so erhaltenen Zwischenprodukt, das gegebenenfalls noch weitere Reservoir- bzw. Matrixschichten und/oder eine die Wirkstofffreisetzung kontrollierende, ebenfalls faserfreie und erforderlichenfalls lichtdurchlässige Membran umfassen kann, werden schließlich durch Schneiden und/oder Stanzen einzelne TTS erzeugt. Im fertigen TTS stellt die Wirkstoff undurchlässige Trägerschicht die ablösbare Schutzschicht dar.

Es ist aber auch möglich, nach dem Aufbringen der wirkstoffhaltigen Zubereitung, jedoch vor dem Ausbringen der Rückschicht, TTS aus dem bis dahin entstandenen Verbundlaminat zu vereinzeln und anschließend mit einer Rückschicht abzudecken.

Das Verfahren zur Herstellung eines, transdermalen therapeutischen Systems zeichnet sich durch die Merkmale des Anspruchs 1 aus

Zur Herstellung des TTS wird der Wirkstoff in einem fließfähigen Zustand benötigt, der eine einzelne Dosierung mittels eines Verteilerplatten-Druckverfahrens ermöglicht. Dies erfolgt bei Wirkstoffen, die bei normalen Herstellungsbedingungen als Feststoff vorliegen, durch Zugabe geeigneter Lösungsmittel sowie gegebenenfalls weiterer Hilfsstoffe, mit denen die Viskosität der Wirkstoffzubereitung eingestellt werden kann. Als Lösungsmittel sind prinzipiell alle gängigen organischen Lösungsmittel geeignet, wie zum Beispiel Ethanol, Isopropylalkohol, Heptan, Hexan, Ethylacetat, Petrolether, Benzin, Aceton, Glycerol, DEET (N,N-Diethyl-3-methylbenzamid),THF sowie viele Öle, beispielsweise Silikonöl, Paraffin, Triglyceride, Neutralöl oder pflanzlich öle.

Flüssige Wirkstoffe können direkt mittel des Druckverfahren aufgetragen werden. Üblicherweise werden jedoch auch flüssige Wirkstoffe in Form einer Flüssigkeit verwendet, die durch Zugabe geeigneter Lösungsmittel und/oder Hilfsstoffe die gewünschte Viskosität aufweist.

Die Viskosität der als Druckmedium zu verwendenden wirkstoffhaltigen Zubereitung liegt vorzugsweise im Bereich von 10 bis 100 dPa·s, besonders bevorzugt in einem Bereich von 15 bis 25 dPa·s.

Indem das Druckverfahren bei möglichst konstanter Temperatur durchgeführt wird, kann eine hohe Dosiergenauigkeit erreicht werden. Vorzugsweise wird das Druckverfahren bei Raumtemperatur durchgeführt, kann aber auch bei niedrigeren oder höheren Temperaturen durchgeführt werden.

Erfindungsgemäß wird der Wirkstoff mit einer Lösung, Suspension oder Dispersion eines Polymers gemischt, das auch Bestandteil der faserfreien Haftkleberschicht für das herzustellende TTS ist. Dabei kann die Viskosität der Mischung durch das Verhältnis von Wirkstoff zu Haftkleber so eingestellt werden, dass die Mischung als Druckmedium verwendet werden kann. Diese Mischung wird dann als Druckmedium verwendet. Bevorzugte Polymere für die Verstellung des Druckmediums sind kationische Copolymere auf Basis von Dimethylaminoethylmethacrylat und neutralen Methacrylsäureestern, beispielsweise Eudragit® E 100, und neutrale Copolymere auf Basis von Butylmethacrylat und Methylmethacrylaten, zum Beispiel Plastoid® B.

Das Verfahren ist besonders gut für Wirkstoffe geeignet, die bei Raumtemperatur flüssig und/oder leicht flüchtig sind, so dass ein Lösen des Wirkstoffs im Kleber und Trocknen bei Temperaturen zwischen 60 und 100 °C nicht machbar ist, weil der Wirkstoff zu leicht flüchtig oder zu thermosensibel ist. Zu diesen Wirkstoffen gehören beispielsweise Nikotin, Nitroglycerin, Bupropion, aber auch ätherische Öle wie Menthol, Kampfer, Terpentin, oder die Öle aus der Latschenkiefer, Pfefferminze, Krauseminze, Zitrone oder Nelken, sowie fast alle Polypeptide und Proteine. Auch DEET und DMSO gehören zu den leichtflüchtigen Substanzen.

Bei einem besonders bevorzugten Verfahren wird Eudragit® E 100 in Nikotin gelöst und diese Wirkstofflösung als Druckmedium verwendet. Das Mengenverhältnis von Nikotin zu Budragit beträgt vorzugsweise 1 : 1 bis 1 : 3, besonders bevorzugt 1,4 : 1 bis 2,4 : 1.

Andere Wirkstoffe, die geeignet sind, um mit dem Verfahren aufgebracht zu werden, sind Alprostadil, Buprenorphin, Clonidin, Dexamothason, Dextroamph-tamin, Diclofenac, Dihydrotestonteron, Estradiol (auch in Kombination mit androgenen oder progestinen Wirkstoffen), Fentanyl, Flurbiprofen, Lidocain, Methylphenidat, Nitroglycerin, Rotigotin, Salicylsäure, Scopolamin, Testosteron und Tulobuterol.

### Vergleichsbeispiel

Eine Haftklebemasse, bestehend aus 2,0825 kg einer 40 %-gen Lösung eines selbstvernetzenden Acrylatpolymers (aus 2-Ethylhxylacrylat, Vinylacetat, Acrylsäure und Titanchelatester; im Handel unter der Bezeichnung Durotak® 280-2416 (National Starch & Chemical B.V) erhältlich in einer Mischung aus Essigsäure-ethylester, Ethanol, Hexan und Menthol, 147 g eines Acrylharzes aus Dimethylaminoethylmethacrylat und neutralen Methacrylsäureestern (Eudragit® E 100 der Firma Röhm-Pharma) sowie 20 g eines gemischt sauren Triglycerids der fraktionierten Kokosfettsäuren C₈-C₁₀ (Miglyol® 812 der Firma Dynamit Nobel) wurden auf eine einseitig bedampfte und beidseitig adhäsiv ausgerüstete Schutzschicht aufgetragen. Anschließend wurde das Lösungsmittel bei 50 bis 80 °C verdampft. Es wurde eine Haftkleberschicht mit einem Flächengewicht von ungefähr 300 g/m² erhalten. Aus der derart hergestellten Haftklebeschicht wurden Ronden mit einem Durchmesser von 65 mm gestanzt, und die überstehenden Ränder wurden abgegittert.

Auf die Haftkleberschicht jeder Ronde wurde eine Dosis von 102 mg Nikotin als Wirkstoff-Zubereitung in Form einer Lösung (140 g Nikotin in 100 g Budragit® B 100) aufgebracht. Dazu wurde ein Tampondrucker mit einer Stahl-Klischeeplatte mit einer runden Vertiefung von 39 mm und einer Ätztiefe von 240 µm als Druckbild zum Bedrucken der Ronden eingesetzt. Als Tampon wurde ein eiförmiger Silikon-Schaumgummitampon mit einer Shore-Härte von 6 eingesetzt. Die Nikotin-Eudragit-Lösung wurde als Druckmedium verwendet, und mittels des Tampons wurden kreisförmige Schichten dieses Druckmedium auf die Ronden gesetzt.

Auf die derart hergestellten "patches" wurde sofort eine transparente, 15 µm dicke Polyesterfolie als nikotinundurchlässige Rückschicht laminiert. Anschließend wurden die fertigen Produkte in Vierrandsiegelbeutel aus bekanntem Verbundpackstoff (Barex®) eingesiegelt.

## Patentansprüche

1. Verfahren zur Herstellung eines transdermalen therapeutischen Systems, **dadurch gekennzeichnet, dass**
- eine wirkstoffundurchlässige Trägerschicht mit einer faserfreien, lichtdurchlässigen Haftkleberschicht versehen wird,
- mittels eines Druckverfahren einzeln dosierte Portionen einer fließfähigen, wirkstoffhaltigen Zubereitung auf die Haftkleberschicht aufgebracht, wobei die fließfähige wirkstoffhaltige Zubereitung ein Polymer umfasst, das auch Bestandteil der faserfreien Haftkleberschicht ist und dass es sich bei dem Druckverfahren um ein Verfahren handelt, bei dem die wirkstoffhaltige Zubereitung durch eine mit mindestens einem Durchlass versehene Verteilerplatte einer Auftragsvorrichtung auf die faserfreie Haftkleberschicht übertragen wird, und
- in einem weiteren Schritt eine lichtdurchlässige, wirkstoffundurchläsige, faserfreie Rückschicht auf die mit der wirkstoffhaltigen Zubereitung versehene Haftkleberschicht aufgebracht wird, wobei TTS durch Schneiden und/oder Stanzen vor oder nach dem Aufbringen der wirkstoffhaltigen Zubereitung aus dem bis dahin entstandenen Verbundlaminat vereinzelt werden können.

2. Verfahren nach einem der vorangehenden Ansprüche, **dadurch gekennzeichnet, dass** das Polymer aus der Gruppe ausgewählt ist, die kationische Copolymere auf Basis von Dimethylaminoethylmethacrylat und neutralen Methacrylsäureestern, und neutrale Copolymere auf Basis von Butylmethacrylat und Methylmethacrylaten umfasst.

3. Verfahren nach einem der vorangehenden Ansprüche, **dadurch gekennzeichnet, dass** die Viskosität der fließfähigen, wirkstoffhaltigen Zubereitung durch Zugabe von Hilfsstoffen eingestellt wird.

4. Verfahren nach einem der vorangehenden Ansprüche, **dadurch gekennzeichnet, dass** die Rückschicht faserfrei ist und aus einem Material besteht, das aus der Gruppe ausgewählt ist, die Polyethylenterephthalat, weichgemachte Vinylacetat-Vinylchlorid-Copolymerisate, Nylon, Ethylen-Vinyl-acetat-Copolymerisate, weichgemachtes Polyvinylchlorid, Polyurethan, Polyvinylidenchlorid, Polypropylen, Polyethylen oder Polyamid umfasst.

5. Verfahren nach einem der vorangehenden Ansprüche, **dadurch gekennzeichnet, dass** die Haftkleberschicht faserfrei ist und ein Material umfasst, das aus der Gruppe ausgewählt ist, die aus haftklebenden Polymeren auf Basis von Acrylsäure und/oder Methacrylsäure sowie deren Estern, Polyacrylaten, Isobutylen, Polyvinylacetat, Ethylen-Vinylacetat, natürlichen und/oder synthetischen Kautschuken, beispielsweise Acrylnitril-Buta-dien-Kautschuk, Butylkautschuk oder Neoprenkautschuk, Styrol-Dien-Co-polymeren wie Styrol-Butadien-Blockcopolymeren und Heißschmelzklebern besteht, oder das auf Basis von haftklebenden Silikonpolymeren oder Polysiloxanen hergestellt ist.

6. Verfahren nach Anspruch 5, **dadurch gekennzeichnet, dass** das Material aus der Gruppe ausgewählt ist, die kationische Copolymere auf Basis von Dimethylaminoethylmethacrylat und neutralen Methacrylsäureestern, und neutrale Copolymere auf Basis von Butylmethacrylat und Methylmethacrylaten umfasst.

7. Verfahren nach einem der vorangehenden Ansprüche, **dadurch gekennzeichnet, dass** der Wirkstoff bei Raumtemperatur flüssig oder ein Feststoff ist.

8. Verfahren nach einem der vorangehenden Ansprüche, **dadurch gekennzeichnet, dass** der Wirkstoff aus der Gruppe ausgewählt ist, die Alprostadil, Buprenorphin, Bupropion Clonidin, Dexamethadon, Dextroamphetamin, Diclofenac, Dihydrotestosteron, Estradiol (auch in Kombination mit androgenen oder progestinen Wirkstoffen), Fentanyl, Flurbiprofen, Lidocain, Methylphenidat, Nikotin, Nitroglycerin, Rotigotin, Salicylsäure, Scopolamin, Testosteron, Tulobuterol und ätherische Öle umfasst.

## Claims

1. Method for producing a transdermal therapeutic system, **characterized in that**
- an active ingredient-impermeable support layer having a fibre-free, light-transmissive layer of pressure-sensitive adhesive is provided,
- individually dispensed portions of a flowable, active ingredient-containing preparation applied to the layer of pressure-sensitive adhesive by means of a printing method, the flowable, active ingredient-containing preparation comprising a polymer which is also a constituent of the fibre-free layer of pressure-sensitive adhesive and the printing method being a method in which the active ingredient-containing preparation is transferred to the fibre-free layer of pressure-sensitive adhesive through a distributor plate of an application device, which distributor plate is provided with at least one conduit, and
- in a further step, a light-transmissive, active ingredient-impermeable, fibre-free backing layer is applied to the layer of pressure-sensitive adhesive provided with the active ingredient-containing preparation, it being possible to singularize TTSs from the hitherto formed composite laminate by cutting and/or punching before or after the application of the active ingredient-containing preparation.

2. Method according to any of the preceding claims, **characterized in that** the polymer is selected from the group comprising cationic copolymers based on dimethylaminoethyl methacrylate and neutral methacrylates and neutral copolymers based on butyl methacrylate and methyl methacrylates.

3. Method according to either of the preceding claims, **characterized in that** the viscosity of the flowable, active ingredient-containing preparation is adjusted by addition of excipients.

4. Method according to any of the preceding claims, **characterized in that** the backing layer is fibre-free and consists of a material which is selected from the group comprising polyethylene terephthalate, plasticized vinyl acetate-vinyl chloride copolymers, nylon, ethylene-vinyl acetate copolymers, plasticized polyvinyl chloride, polyurethane, polyvinylidene chloride, polypropylene, polyethylene or polyamide.

5. Method according to any of the preceding claims, **characterized in that** the layer of pressure-sensitive adhesive is fibre-free and comprises a material which is selected from the group consisting of pressure-sensitive adhesive polymers based on acrylic acid and/or methacrylic acid and also the esters thereof, polyacrylates, isobutylene, polyvinyl acetate, ethylene-vinyl acetate, natural and/or synthetic rubbers, for example acrylonitrile-butadiene rubber, butyl rubber or neoprene rubber, styrene-diene copolymers such as styrene-butadiene block copolymers and hot-melt adhesives or which has been produced on the basis of pressure-sensitive adhesive silicone polymers or polysiloxanes.

6. Method according to Claim 5, **characterized in that** the material is selected from the group comprising cationic copolymers based on dimethylaminoethyl methacrylate and neutral methacrylates and neutral copolymers based on butyl methacrylate and methyl methacrylates.

7. Method according to any of the preceding claims, **characterized in that** the active ingredient is liquid or a solid at room temperature.

8. Method according to any of the preceding claims, **characterized in that** the active ingredient is selected from the group comprising alprostadil, buprenorphine, bupropion clonidine, dexamethadone, dextroamphetamine, diclofenac, dihydrotestosterone, oestradiol (including in combination with androgenic or progestinal active ingredients), fentanyl, flurbiprofen, lidocaine, methylphenidate, nicotine, nitroglycerin, rotigotine, salicylic acid, scopolamine, testosterone, tulobuterol and essential oils.

## Revendications

1. Procédé pour la fabrication d'un système thérapeutique transdermique **caractérisé en ce que**
- on munit une couche de support imperméable aux substances actives d'une couche auto-adhésive translucide exempte de fibres,
- au moyen d'un procédé d'impression, on applique, sur la couche auto-adhésive, des portions dosées de manière individuelle d'une préparation à écoulement libre contenant une ou plusieurs substances actives, dans lequel la préparation à écoulement libre contient une ou plusieurs substances actives comprenant un polymère qui représente également un constituant de la couche auto-adhésive exempte de fibres et **en ce qu'**il s'agit, en ce qui concerne le procédé d'impression, d'un procédé dans lequel la préparation contenant une ou plusieurs substances actives est transférée sur la couche auto-adhésive exempte de fibres via une plaque de répartition d'un dispositif d'application, munie au moins d'un passage, et
- dans une étape ultérieure, on applique, sur la couche auto-adhésive munie de la préparation contenant une ou plusieurs substances actives, une couche dorsale exempte de fibres, translucide, imperméable aux substances actives, dans lequel on peut isoler des TTS par découpe et/ou par estampage avant ou après l'application de la préparation contenant une ou plusieurs substances actives, à partir du stratifié composite obtenu entre-temps.

2. Procédé selon l'une quelconque des revendications précédentes, **caractérisé en ce que** le polymère est sélectionné à partir du groupe qui comprend des copolymères cationiques à base de diméthacrylate de diméthylaminoéthyle et des esters neutres d'acide méthacrylique, et des copolymères neutres à base de méthacrylate de butyle et de méthacrylates de méthyle.

3. Procédé selon l'une quelconque des revendications précédentes, **caractérisé en ce qu'**on règle la viscosité de la préparation à écoulement libre contenant une ou plusieurs substances actives par addition de substances auxiliaires.

4. Procédé selon l'une quelconque des revendications précédentes, **caractérisé en ce que** la couche dorsale est exempte de fibres et est constituée d'une matière qui est sélectionnée à partir du groupe qui comprend du polyéthylène téréphtalate, des copolymères plastifiés d'acétate de vinyle-chlorure de vinyle, du nylon, des copolymères d'éthylène-acétate de vinyle, du chlorure de polyvinyle plastifié, du polyuréthane, du chlorure de polyvinylidène, du polypropylène, du polyéthylène ou du polyamide.

5. Procédé selon l'une quelconque des revendications précédentes, **caractérisé en ce que** la couche auto-adhésive est exempte de fibres et comprend une matière qui est choisie parmi le groupe qui est constitué par des polymères auto-adhésifs à base d'acide acrylique et/ou à base d'acide méthacrylique et de leurs esters, des polyacrylates, de l'isobutylène, de l'acétate de polyvinyle, de l'éthylène-acétate de vinyle, des caoutchouc naturels et/ou synthétiques, par exemple du caoutchouc d'acrylonitrile-butadiène, du caoutchouc butyle ou du caoutchouc de néoprène, des copolymères de styrène-diène, tels que des copolymères séquencés de styrène-butadiène et des adhésifs thermofusibles, ou bien que l'on prépare sur la base de polysiloxanes ou de polymères de silicone auto-adhésifs.

6. Procédé selon la revendication 5, **caractérisé en ce que** la matière est sélectionnée à partir du groupe qui comprend des copolymères cationiques à base de méthacrylate de diméthylaminoéthyle et d'esters neutres d'acide méthacrylique, et des copolymères neutres à base de méthacrylate de butyle et de méthacrylates de méthyle.

7. Procédé selon l'une quelconque des revendications précédentes, **caractérisé en ce que** la substance active est liquide à la température ambiante ou représente une substance solide.

8. Procédé selon l'une quelconque des revendications précédentes, **caractérisé en ce que** la substance active est sélectionnée à partir du groupe qui comprend l'alprostadil, la buprénorphine, le bupropion la clonidine, la dexaméthasone, la dextro-amphétamine, le diclofenac, la dihydrotestostérone, l'estradiol (également en combinaison avec des substances actives androgènes ou progestatives), le fentanyle, le flurbiprofène, la lidocaïne, le méthylphénidate, la nicotine, la nitroglycérine, la rotigodine, l'acide salicylique, la scopolamine, la testostérone, le tulobutérol, et des huiles éthérées.
